# EUROPEAN PATENT SPECIFICATION

(11) **EP 2 970 860 B1**
(45) Date of publication and mention of the grant of the patent: **21.02.2018**
(21) Application number: 14775282.8
(22) Date of filing: 26.02.2014
(51) Int. Cl.: C12N 1/21, C07K 14/195, C12N 9/04, C12N 9/88, C12N 9/90

(54) **GENETICALLY MODIFIED BACTERIA**
GENTECHNISCH VERÄNDERTE BAKTERIEN
BACTÉRIES MODIFIÉES GÉNÉTIQUEMENT

(30) Priority: 14.03.2013 US 201361782141 P
(43) Date of publication of application: 20.01.2016
(73) Proprietor: President and Fellows of Harvard College, Cambridge, MA 02138 (US)
(72) Inventor: MARX, Christopher J., Moscow, Idaho 83843 (US); NAYAK, Dipti D., Urbana, Illinois 61801-3263 (US)
(74) Representative: Regimbeau
(86) International application number: PCT/US2014/018592
(87) International publication number: WO 2014/158590

(56) References cited:
- WO-A2-2012/088071
- US-A1- 2012 052 542
- HIROYA YURIMOTO ET AL: "Genomic organization and biochemistry of the ribulose monophosphate pathway and its application in biotechnology", APPLIED MICROBIOLOGY AND BIOTECHNOLOGY, SPRINGER, BERLIN, DE, vol. 84, no. 3, 11 July 2009 (2009-07-11), pages 407-416, XP019737781, ISSN: 1432-0614, DOI: 10.1007/S00253-009-2120-7
- CHISTOSERDOVA, L ET AL.: 'The Expanding World Of Methylotrophic Metabolism.' ANNUAL REVIEW OF MICROBIOLOGY. vol. 63, no. 1, 01 January 2009, pages 477 - 499, XP055047826 DOI: 10.1146/ANNUREV.MICRO.091208.073600

## Description

### FIELD

The present invention relates in general to genetically modified bacteria, such as *Methylobacterium,* having an increased biomass producing capability. Such genetically modified bacteria can be used to produce useful chemical compounds from single-carbon (C₁) compounds such as methanol (CH₃OH) and methane (CH₄).

### BACKGROUND

Methylotrophy is the ability of microorganisms, such as bacteria and certain fungi, to grow on reduced C₁ compounds like CH₄ and CH₃OH as the sole carbon and energy source. Non-methylotrophic bacteria, such as Pseudomonas putida and Escherichia coli, have been genetically engineered to utilize methanol and formaldehyde as substrate by introduction of genes from C1 assimilation pathways (Yurimoto H. et al., Appl Microbiol Biotechnol. 2009 Sep; 84(3):407-16; US 2012/0052542). As some C₁ assimilation pathways are highly inefficient, metabolic pathways diverting less feedstock to carbon dioxide (CO₂) would be useful for production of beneficial carbon-based compounds. Therefore, genetically modified microorganisms optimized to reduce CO₂ waste are desirable.

### SUMMARY

Embodiments of the present disclosure are directed to the modification of pathways within bacteria to increase overall retention of carbon in central metabolism versus obligate waste to CO₂ when grown on C₁ compounds such as methanol. According to one aspect, genetically modified bacteria described herein have an increased substrate yield (grams carbon into biomass vs. grams carbon to CO₂) compared to bacteria without the genetic modifications, including wild-type bacteria. In this aspect, biomass production is increased per unit substrate due to the increased efficiency of the genetically modified bacteria. According to an alternate aspect, genetically modified bacteria described herein produce less carbon dioxide and more carbon-based biomass compared to bacteria without the genetic modification, including wild-type bacteria. In this aspect, biomass production is increased per substrate.

According to certain aspects of the present disclosure, a recombinant bacterium is provided which has been genetically modified to disrupt the bacterium's H₄MPT (tetrahydromethanopterin) pathway for dissimilation of C₁ compounds like CH₃OH. According to this aspect, one or more genes within the bacterium's H₄MPT (tetrahydromethanopterin) pathway for C₁ dissimilation are removed, altered, inhibitor-bound, or otherwise prevented from being expressed. In this manner, the bacterium's H₄MPT pathway for C₁ dissimilation is disrupted, inhibited, silenced or otherwise rendered ineffective for C₁ dissimilation. According to this aspect, C₁ dissimilation need not be completely prevented by disruption of the H₄MPT pathway. Instead, genetic modification may result in some operation of the pathway, however, the genetic modification is intended to disable the H₄MPT pathway relative to the unmodified H₄MPT pathway.

According to certain aspects of the present disclosure, a recombinant bacterium is provided which has been genetically modified to disrupt the bacteria's serine cycle for lower carbon assimilation, such as C₁ assimilation. According to this aspect, one or more genes within the bacterium's serine cycle for C₁ assimilation are removed, altered, inhibitor-bound, or otherwise prevented from being expressed. In this manner, the bacterium's serine cycle for C₁ assimilation is disrupted, inhibited, silenced or otherwise rendered ineffective for C₁ assimilation. According to this aspect, C₁ assimilation need not be completely prevented by disruption of the serine cycle. Instead, genetic modification may result in some operation of the serine cycle, however, the genetic modification is intended to disable the serine cycle relative to the unmodified serine cycle.

According to certain aspects of the present disclosure, a bacterium may be genetically modified to disrupt the bacterium's H₄MPT pathway for C₁ dissimilation or to disrupt the bacterium's serine cycle for C₁ assimilation. According to certain aspects of the present disclosure, a bacterium may be genetically modified to disrupt the bacterium's H₄MPT pathway for C₁ dissimilation and to disrupt the bacterium's serine cycle for C₁ assimilation.

According to certain aspects, a bacterium may be genetically modified to include one or more genes from a RuMP (ribulose monophosphate) pathway for C₁ assimilation and C₁ dissimilation. One or more genes from the RuMP pathway encode for certain C₁ assimilation enzymes and when included into the bacterium, the enzymes are expressed to carry out C₁ assimilation. One or more genes from the RuMP pathway encode for certain C₁ dissimilation enzymes and when included into the bacterium, the enzymes are expressed to carry out C₁ dissimilation. It is to be understood that not all genes attributed by those of skill in the art to the RuMP pathway need to be included into the genome of a bacteria to have the bacteria express the enzymes responsible for either C₁ assimilation or C₁ dissimilation.

According to one aspect, a bacterium may be genetically modified to disrupt the bacterium's H₄MPT pathway for C₁ dissimilation and to include one or more genes from a RuMP pathway for C₁ assimilation and C₁ dissimilation. According to a further aspect, a bacterium may be genetically modified to disrupt the bacterium's serine cycle for C₁ assimilation and to include one or more genes from an RuMP pathway for C₁ assimilation and C₁ dissimilation. According to a still further aspect, a bacterium may be genetically modified to disrupt the bacterium's H₄MPT pathway for C₁ dissimilation and to disrupt the bacterium's serine cycle for C₁ assimilation and to include one or more genes from an RuMP pathway for C₁ assimilation and C₁ dissimilation.

According to certain aspects, the bacteria may be any which contain the serine cycle or the Calvin Cycle for C₁ assimilation, and either the H₄MPT or thiol-based (e.g., glutathione, mycothiol) C₁ dissimilation pathway. According to the present disclosure, such bacteria may be improved by disabling the serine cycle, the Calvin Cycle, the H₄MPT C₁ dissimilation pathway or the thiol-based (e.g., glutathione, mycothiol) C₁ dissimilation pathway and enabling the RuMP pathway for either C₁ assimilation, C₁ dissimilation or both. According to certain aspects, the bacteria may be a *Methylobacterium.* According to certain aspects, the bacteria may be a *Methylobacterium extorquens.* According to certain aspects, the bacteria may be a *Methylobacterium extorquens* AMI. According to certain aspects, the bacteria may be a *Methylobacterium extorquens* PA1.

According to one aspect, a *Methylobacterium extorquens* PA1 cell is genetically modified to disrupt the cell's H₄MPT (tetrahydromethanopterin) pathway for C₁ dissimilation and to include a recombinant RuMP pathway for C₁ dissimilation.

According to an alternate aspect, a *Methylobacterium extorquens* PA1 cell is genetically modified to disrupt the cell's serine cycle for C₁ assimilation and to include a recombinant RuMP pathway for C₁ assimilation.

According to an alternate aspect, a *Methylobacterium extorquens* PA1 cell is genetically modified to disrupt the cell's H₄MPT (tetrahydromethanopterin) pathway for C₁ dissimilation, to disrupt the cell's serine cycle for C₁ assimilation and to include a recombinant RuMP pathway for C₁ assimilation and C₁ dissimilation.

### BRIEF DESCRIPTION OF THE DRAWINGS

The foregoing and other features and advantages of the present invention will be more fully understood from the following detailed description of illustrative embodiments taken in conjunction with the accompanying drawing in which:

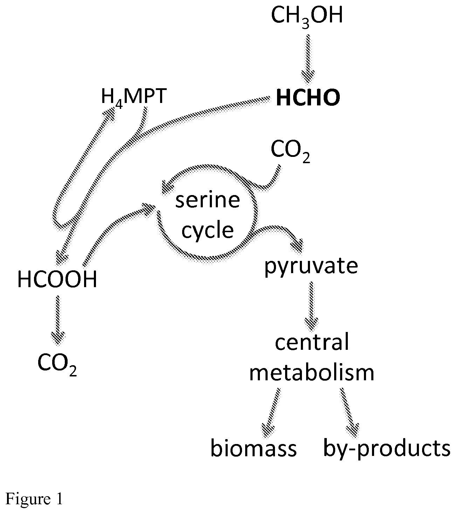

Figure 1 is the metabolic pathways present in an unaltered serine cycle methylotroph. Native pathways contributing to both C₁ assimilation and dissimilation are shown in purple; pathways solely for C₁ dissimilation in red; pathways for C₁ assimilation in turquoise; pathways of central metabolism in grey.

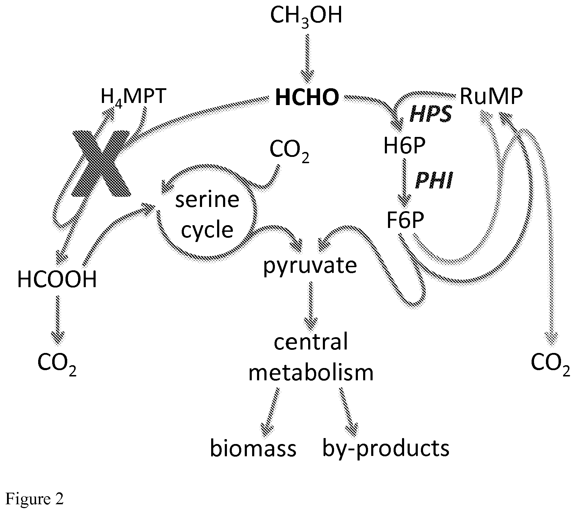

Figure 2 is the metabolic pathways present in a serine cycle methylotroph in which the H₄MPT-dependent formaldehyde oxidation pathway has been disabled (bright red X), and an RuMP cycle has been introduced, such as through the introduction and expression of HPS and PHI (dark blue). These introduce novel reactions for C₁ assimilation and dissimilation (brown), novel C₁ dissimilation in orange, and novel C₁ assimilation in green. Remaining colors are as described in Figure 1.

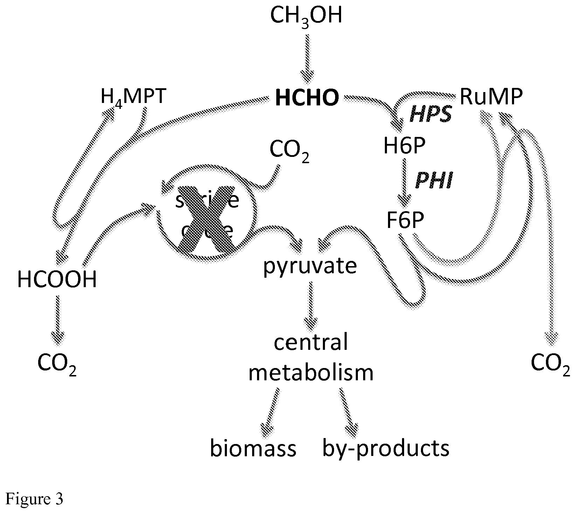

Figure 3 is the metabolic pathways present in a serine cycle methylotroph in which the native serine cycle for C₁ assimilation has been disabled (bright red X), and an RuMP cycle has been introduced, such as through the introduction and expression of HPS and PHI. Remaining colors are described in Figures 1 and 2.

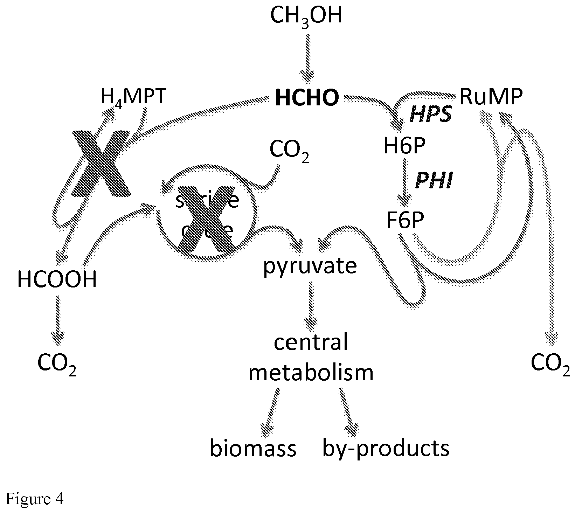

Figure 4 is the metabolic pathways present in a serine cycle methylotroph in which the H₄MPT-dependent formaldehyde oxidation pathway and the serine cycle have been disabled (bright red Xs), eliminating both C₁ assimilation and dissimilation functions, and an RuMP cycle has been introduced, such as through the introduction and expression of HPS and PHI. Remaining colors are described in Figures 1 and 2.

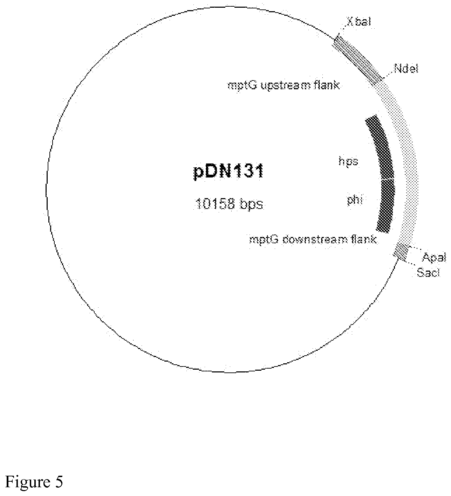

Figure 5 shows the plasmid map of the construct made to knockout *hprA* in the serine cycle and to replace it with the genes encoding hexulose 6-phosphate synthase (HPS) and hexulose 6-phosphate isomerase (PHI) from *Methylococcus capsulatus* Bath. The construct was made on the backbone of pCM433 [*GenBank:*EU118176] which is a 8081 bp long Cam^{R}, Tet^{R}, Amp^{R} vector with a ColE1 origin of replication and an IncP origin of transfer (Marx, BMC Research Notes 1:1 (2008)).

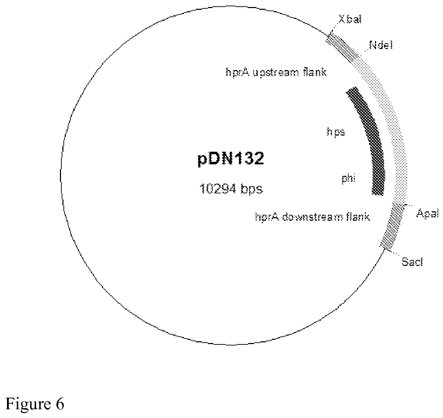

Figure 6 shows the plasmid map of the construct made to knockout *hprA* in the serine cycle and to replace it with the genes encoding hexulose 6-phosphate synthase (HPS) and hexulose 6-phosphate isomerase (PHI) from *Methylococcus capsulatus* Bath. The construct was made on the backbone of pCM433 [*GenBank:*EU118176] which is a 8081 bp long Cam^{R}, Tet^{R}, Amp^{R} vector with a ColE1 origin of replication and an IncP origin of transfer (Marx, BMC Research Notes 1:1 (2008)).

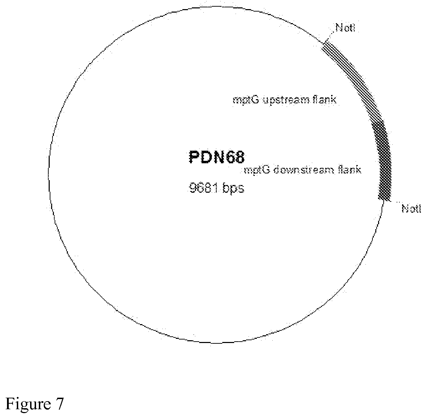

Figure 7 shows the plasmid map of the construct made to knockout *mptG* in *M. extorquens* PA1. The construct was made on the backbone of pCM433 [*GenBank:*EU118176] which is a 8081 bp long Cam^{R}, Tet^{R}, Amp^{R} vector with a ColE1 origin of replication and an IncP origin of transfer (Marx, BMC Research Notes 1:1 (2008)).

### DETAILED DESCRIPTION

Embodiments of the present disclosure include a recombinant host microorganism that includes one or more genetic modifications which result in reduced carbon dioxide production and concomitant increase in flow to biomass or by-product production. Embodiments of the present disclosure include a recombinant host microorganism that includes one or more genetic modifications that result in increased rate of cell proliferation. Embodiments of the present disclosure include a recombinant host microorganism that includes one or more genetic modifications that result in increased biomass production per substrate.

Embodiments of the present disclosure include recombinant host microorganisms described herein that have been further genetically modified to include a biosynthetic pathway for a target carbon-containing compound. According to one aspect, the recombinant host microorganisms have been genetically modified to reduce use of a carbon feedstock to produce carbon dioxide resulting in an increased production of carbon biomass by the recombinant host microorganisms. When a recombinant host microorganism has been further genetically modified to include a biosynthetic pathway for a target carbon-containing compound, the recombinant host microorganism produces the target compound in a greater amount compared to a host organism that has not been recombinantly modified as described herein.

Aspects of the present disclosure relate to recombinant methylotrophic microorganisms being genetically modified to increase overall biomass production from lower carbon-containing compounds, such as C₁ compounds. Methylotrophic microorganisms are those, such as bacteria and certain fungi, which grow on C₁ compounds like CH₄ and CH₃OH as the carbon and energy source. Methylotrophic microorganisms may also include those which, in addition to C₁ compounds, grow on lower carbon compounds such as C₂ to C₅ carbon compounds.

Standard recombinant DNA and molecular cloning techniques used herein are well known in the art and are described in Sambrook, J., Fritsch, E.F. and Maniatis, T., Molecular Cloning: A Laboratory Manual, 2nd ed.; Cold Spring Harbor Laboratory: Cold Spring Harbor, N.Y., (1989) and by Silhavy, T.J., Bennan, M.L. and Enquist, L.W., Experiments with Gene Fusions; Cold Spring Harbor Laboratory: Cold Spring Harbor, N.Y., (1984); and by Ausubel, F.M. et. al., Current Protocols in Molecular Biology, Greene Publishing and Wiley-Interscience (1987) each of which are hereby incorporated by reference in their entireties.

Additional useful methods are described in manuals including Advanced Bacterial Genetics (Davis, Roth and Botstein, Cold Spring Harbor Laboratory, 1980), Experiments with Gene Fusions (Silhavy, Berman and Enquist, Cold Spring Harbor Laboratory, 1984), Experiments in Molecular Genetics (Miller, Cold Spring Harbor Laboratory, 1972) Experimental Techniques in Bacterial Genetics (Maloy, in Jones and Bartlett, 1990), and A Short Course in Bacterial Genetics (Miller, Cold Spring Harbor Laboratory 1992) each of which are hereby incorporated by reference in their entireties.

Methylotropic microorganisms may be genetically modified to delete genes or incorporate genes by methods known to those of skill in the art. Genes within the methylotropic microorganisms to be inhibited are known to those of skill in the art or may be determined using methods known to those of skill in the art. Genes, or homologs thereof, to be added to the genome of the methylotropic microorganisms are known to those of skill in the art or may be identified and obtained using methods known to those of skill in the art. Vectors and plasmids useful for transformation of some host cells may be common and commercially available from companies such as Invitrogen Corp. (Carlsbad, CA), Stratagene (La Jolla, CA) and New England Biolabs, Inc. (Beverly, MA). Other organisms will require broad-host-range vectors such as the pCM series generated from a minimal IncP replicon (Marx and Lidstrom, Microbiology 147-2065-2075 (2001)), or that take advantage of conjugal transfer to introduce suicide vectors (Marx and Lidstrom, BioTechniques 33:1062-1067 (2002); Marx, BMC Research Notes 1:1 (2008)).

Typically, the vector or plasmid contains sequences directing transcription and translation of a relevant gene, a selectable marker, and sequences allowing autonomous replication or chromosomal integration. Suitable vectors comprise a region 5' of the gene which harbors transcriptional initiation controls and a region 3' of the DNA fragment which controls transcription termination. Both control regions may be derived from genes homologous to the transformed host cell, although it is to be understood that such control regions may also be derived from genes that are not native to the species chosen as a production host.

Initiation control regions or promoters, which are useful to drive expression of the relevant pathway coding regions in the desired host cell are numerous and familiar to those skilled in the art. Virtually any promoter capable of driving these genetic elements is suitable for the present invention including, but not limited to, *lac, ara, tet, trp, IP_{L}, IP_{R}, T7, tac,* and *trc* (useful for expression in *Escherichia coli* and *Pseudomonas*); or *mxaF,* and *tacA* for *Methylobacterium* spp. (Marx and Lidstrom, Microbiology 147:2065-2075 (2001); Chou et al., PLoS Genet. 5:e1000652 (2009)).. Termination control regions may also be derived from various genes native to the preferred hosts, or introduced from *E. coli,* such as t*_{rrnB}* or t*_{T7}* (Marx and Lidstrom, Microbiology 150:9-19 (2004)).

Certain vectors are capable of replicating in a broad range of host bacteria and can be transferred by conjugation. The complete and annotated sequence of pRK404 and three related vectors-pRK437, pRK442, and pRK442(H) are available. These derivatives have proven to be valuable tools for genetic manipulation in Gram-negative bacteria (Scott et al., Plasmid 50(1):74-79 (2003)). Several plasmid derivatives of broad-host-range Inc P4 plasmid RSF1010 are also available with promoters that can function in a range of Gram-negative bacteria. Plasmid pAYC36 and pAYC37, have active promoters along with multiple cloning sites to allow for the heterologous gene expression in Gram-negative bacteria.

Vectors useful for the transformation of *E. coli* are common and commercially available. For example, the desired genes may be isolated from various sources, cloned onto a modified pUC19 vector and transformed into *E. coli* host cells. Alternatively, the genes encoding a desired biosynthetic pathway may be divided into multiple operons, cloned onto expression vectors, and transformed into various *E. coli* strains.

The various genes for a desired biosynthetic or other desired pathway may be assembled into any suitable vector, such as those described above. The codons can be optimized for expression based on the codon index deduced from the genome sequences of the host strain, such as for *M. extorquens* (Agashe et al., Mol. Biol. Evol. 30:549-560 (2013)).

Methylotrophic microorganisms which may serve as host cells and which may be genetically modified to produce recombinant methylotrophic microorganisms include methylotrophic bacteria and methylotrophic fungi.

Methylotrophic microorganisms which may serve as host cells and which may be genetically modified to produce recombinant methylotrophic microorganisms as described herein may include one or members of the genera *Clostridium, Rhodobacter, Xanthobacter, Brevibacterium, Mycobacterium, Amycolaptosis, Bacillus, Methylobacillus, Methylomicrobium, Methylotenera, Paracoccus, Methylocella, Methylacidiphium, Methylobacterium, Methylococcus, Methylobacter, Methylibium, Leisingera, Methylophilus, Methylosulfonomonas, Hyphomicrobium, Methylocystis, Methylosinus, Methylomirabilis, Methylophaga* and *Methyloversatilis.*

Methylotrophic microorganisms which may serve as host cells and which may be genetically modified to produce recombinant methylotrophic microorganisms as described herein include bacteria including the serine cycle or the Calvin cycle for C₁ assimilation or the H₄MPT pathway or the thiol-based (e.g., glutathione, mycothiol) C₁ dissimilation pathway.

Methylotrophic microorganisms which may serve as host cells and which may be genetically modified to produce recombinant methylotrophic microorganisms as described herein may include *Methylobacterium spp.* such as *M. extorquens* AMI, *M. extorquens* PA1, *M. extorquens* DM4, *M. extorquens* CM4, *M. populi* BJ001, *M. nodulans* ORS 2060, *Methylobacterium* spp. 4-46, *M. radiotolerans* JCM 2831, *Paracoccus denitrificans* PD122, *Xanthobacter autotrophicus* Py2, and *Hyphomicrobium denitrificans.*

*Methylobacterium extorquens* AMI is a well-characterized bacterium and is described in (Peel and Quayle, Biochem. Journal 81(3): 465-469 (1961)). The genes encoding enzymes involved in C₁ growth of *Methylobacterium extorquens* AMI are described in (Chistoserdova et al., J. Bacteriol. 185(10): 2980-2987 (2003)).

*Methylobacterium extorquens* PA1 is a well-characterized bacterium and is described in (Knief et al., Microb. Ecol. 60: 440-452 (2010)). The genes encoding enzymes involved in C₁ growth of *Methylobacterium extorquens* PA1 are described in (Marx et al., J. Bacteriol. 194: 4746-4748 (2012)).

According to certain aspects, a carbon-containing compound is used as a feedstock or growth substrate for the recombinant microorganisms described herein. Suitable carbon containing compounds useful as a feedstock include any C₁ compounds, or multi-C compounds that contain C₁ units such as methylated amines, methylated sulfhydryls, methoxy groups, etc. Such carbon-containing compounds include methane, methanol, methylamine hydrochloride, sodium formate, betaine, sarcosine, methanethiol, and formaldehyde. Exemplary carbon-containing compounds useful as a feedstock include methane and methanol.

According to certain aspects of the present disclosure a *Methylobacterium,* such as *M. extorquens* PA1, is genetically modified to produce a recombinant *Methylobacterium,* such as a recombinant *M. extorquens* PA1 cell. According to certain aspects, a *Methylobacterium* is genetically modified to disrupt the bacterial cell's H₄MPT pathway for C₁ dissimilation. The bacterial cell's H₄MPT pathway for C₁ dissimilation is well characterized as provided in (Marx et al., J. Bacteriol. 185:7160-7168 (2003)). A summary of the genes and proteins characterizing the H₄MPT pathway for C₁ dissimilation is detailed in the text below and shown in Figure 1.

According to this aspect, disruption of the cell's H₄MPT pathway for C₁ dissimilation includes altering the pathway to alter or otherwise eliminate one or more genes within the pathway in a manner to prevent C₁ dissimilation by the pathway. One or more genes within the H₄MPT pathway for C₁ dissimilation that can be altered or otherwise eliminated include *fae, mtdB, fhcABCD, mptG, mch, dmrA,* ORF5, ORF7, ORF9, ORF17, ORF19, ORF20, ORF21, ORF22, and ORFY. According to one aspect, one or more genes within the H₄MPT pathway for C₁ dissimilation can be altered or otherwise eliminated using methods known to those of skill in the art. For example, such methods include cloning the upstream and downstream flanks of the gene to be deleted, altered, or modified by PCR, restriction enzyme mediated DNA digestion, and subsequent DNA ligation, in an allelic exchange vector that cannot replicate in the host strain; introducing the allelic exchange vector in the host through a tri-parental mating using a pRK2073 containing helper strain as described in Marx, BMC Research Notes, 1:1 (2008) hereby incorporated by reference in its entirety for all purposes.

According to certain aspects of the present disclosure a *Methylobacterium,* such as *M. extorquens* PA1, is genetically modified to produce a recombinant *Methylobacterium,* such as a recombinant *M. extorquens* PA1 cell. According to certain aspects, a *Methylobacterium* is genetically modified to disrupt the bacterial cell's serine cycle for C₁ assimilation. The bacterial cell's serine cycle for C₁ assimilation is well characterized as provided in (Chistoserdova et al., J. Bacteriol. 185(10): 2980-2987 (2003)). A summary of the genes and proteins characterizing the serine cycle for C₁ assimilation is detailed in the text below and shown in Figure 1.

According to this aspect, disruption of the cell's serine cycle for C₁ assimilation includes altering the pathway to alter or otherwise eliminate one or more genes within the pathway in a manner to prevent C₁ assimilation by the pathway. One or more genes within the serine cycle for C₁ assimilation that can be altered or otherwise eliminated include *glyA, hprA, sga, glc, eno, ppc, mdh,* and *mtkAB.* According to one aspect, one or more genes within the serine cycle for C₁ assimilation can be altered or otherwise eliminated using methods known to those of skill in the art. For example, such methods include cloning the upstream and downstream flanks of the gene to be deleted, altered, or modified by PCR, restriction enzyme mediated DNA digestion, and subsequent DNA ligation, in an allelic exchange vector that cannot replicate in the host strain; introducing the allelic exchange vector in the host through a tri-parental mating using a pRK2073 containing helper strain as described in Marx, BMC Research Notes, 1:1 (2008)

According to certain aspects of the present disclosure a *Methylobacterium,* such as *M. extorquens* PA1, is genetically modified to produce a recombinant *Methylobacterium,* such as a recombinant *M. extorquens* PA1 cell. According to certain aspects, a *Methylobacterium* is genetically modified to include one or more genes to express proteins and enzymes responsible for C₁ assimilation and C₁ dissimilation. The genes (HPS and PHI) that need to be incorporated encode two enzymes (hexulose 6-phosphate synthase and hexulose 6-phosphate isomerase) that catalyze the first two steps of the RuMP pathway. While these two genes are generally found as two separate ORFs (Open Reading Frames) in methylotrophs using the RuMP pathway for C₁ assimilation and /or C₁ dissimilation, certain methylotrophs also use a single enzyme encoding a fused polypeptide with two domains - one with hexulose 6-phosphate synthase activity and the other with a hexulose 6-phosphate isomerase activity in the RuMP pathway for C₁ assimilation and/or C₁ dissimilation. According to one aspect, genes encoding the RuMP pathway for C₁ assimilation and C₁ dissimilation and inserted into the genome of the *Methylobacterium.* The RuMP pathway for C₁ assimilation and C₁ dissimilation is well characterized as provided in (Ward et al., PLoS Biology 2:e303 (2004)). A summary of the genes and proteins characterizing the RuMP pathway for C₁ assimilation and C₁ dissimilation is provided in Table 3 below.

| Genes/Enzymes | Function |
|---|---|
| Hexulose 6-phosphate synthase (HPS) | Catalyzes the reaction of ribulose monophosphate and formaldehyde to hexulose 6-phosphate |
| Hexulose 6-phosphate isomerase (PHI) | Catalyzes the isomerization of hexulose 6-phosphate to fructose 6-phosphate |
| Phosphofructokinase (PFK) | Catalyzes the phosphorylation of fructose 6-phosphate to fructose 1,6-bisphosphate |
| Fructose bisphosphate aldolase (FBA) | Catalyzes the cleavage of fructose 1,6-bisphosphate to dihydroxyacetone 3-phosphate and glyceraldehyde 3-phosphate |
| Transketolase (TK) | Catalyzes the rearrangement of C₃-C₇ phosphorylated sugars |
| Pentose phosphate epimerase | Catalyzes the epimerization of xylulose 5-phosphate to ribulose 5-phoshpahte |
| Pentose phosphate isomerase | Catalyzes the isomerization of ribose 5-phosphate to ribulose 5-phoshpahte |
| Glucose phosphate isomerase (GPI) | Catalyzes the isomerization of fructose 6-phosphate to glucose 6-phoshpahte |
| Glucose 6-phosphate dehydrogenase | Catalyzes the oxidation of glucose 6-phosphate to 6-phosphogluconate |
| Phosphogluconate dehydrase | Catalyzes the dehydration of 6-phosphogluconate to 2-keto, 3-deoxy, 6-phosphogluconate (KDPG) |
| 2-keto, 3-deoxy, 6-phosphogluconate (KDPG) aldolase | Catalyzes the cleavage of 2-keto, 3-deoxy, 6-phosphogluconate (KDPG) to glyceraldehyde 3-phosphate and pyruvate |
| Sedoheptulose bisphosphatase | Catalyzes the dephosphorylation of sedoheptulose 1,7-bisphosphate to sedoheptulose 7-phosphate |
| Phosphogluconate dehydrogenase | Catalyzes the decarboxylation of 6-phosphogluconate to ribulose 5-phosphate |

According to this aspect, insertion of the RuMP pathway for C₁ assimilation and C₁ dissimilation into the genome of the *Methylobacterium* includes inserting one or more genes within the pathway in a manner to promote C₁ assimilation and C₁ dissimilation. One or more genes within the RuMP pathway for C₁ assimilation and C₁ dissimilation that can promote C₁ assimilation and C₁ dissimilation include a gene (HPS) encoding a hexulose 6-phosphate synthase and a gene (PHI) encoding a hexulose 6-phosphate isomerase or a gene encoding a fusion polypeptide containing a hexulose 6-phosphate synthase domain as well as a hexulose 6-phosphate isomerase domain. According to one aspect, one or more genes within the RuMP pathway for C₁ assimilation and C₁ dissimilation can be inserted within the genome of the *Methylobacterium* using methods known to those of skill in the art. For example, such methods include cloning the promoter, RBS (Ribosome Binding Site) and entire ORF (Open Reading Frame) as well as the transcription termination site of the genes to be inserted by PCR, restriction enzyme mediated DNA digestion, and subsequent DNA ligation, in an allelic exchange vector that cannot replicate in the host strain; introducing the allelic exchange vector in the host through a tri-parental mating using a pRK2073 containing helper strain as described in Marx, BMC Research Notes, 1:1 (2008).

According to certain aspects, recombinant host microorganisms described herein have been further genetically modified to include a biosynthetic pathway for a target carbon-containing compound. Certain useful target carbon-containing compounds include but are not limited to polyhydroxyalkanoates and related storage polymers (Borque et al., Appl. Microbiol. Biotechnol. 44:367-376 (1995)), polysaccharides (Oh et al., Biotechnol. Bioeng. 54:115-121 (1997)), amino acids such as serine (Sirirote et al., J. Fermen. Technol. 66:291-297 (1988)), liquid biofuels such as ethanol and butanol, 1,4-butanediol, isoprenoids (Van Dien et al., Appl. Environ. Microbiol. 69:7563-7566 (2003)), and plant growth hormones such as indole-3-acetic acid (Omer et al., Plant Growth Regul. 43:93-96 (2004)) *trans-*zeatin (Koenig et al., J. Bacteriol. 184:1832-1842 (2002)). Furthermore, C₁-based protein production could be enhanced due to higher biomass efficiencies for active polypeptides such as enterocin P (Gutierrez et al., FEMS Microbiol. Lett. 248:125-131 (2005)), haloalkane dehalogenase (FitzGerald and Lidstrom, Biotechnol. Bioeng. 81:263-268 (2003)), and esterase (Choi et al., Appl. Environ. Microbiol. 72:753-759 (2006)).

Biosynthetic pathways for useful target carbon-containing compounds are known to those of skill in the art and include the pathway for the generation and incorporation of β-hydroxybutyrl-CoA into polyhydroxybutyrate or related polymers (Korotkova and Lidstrom, J. Bacteriol. 184:1750-1758 (2001)), the biosynthetic pathway for conversion of isopentyl pyrophosphate into carotenoids (Van Dien et al., Appl. Environ. Microbiol. 69:7563-7566 (2003)), the isopentylation of adenines of some tRNAs by the *miaA* gene product to produce trans-zeatin (Koenig et al., J. Bacteriol. 184:1832-1842 (2002)), the pathway for indole-3-acetic acid from tryptophan (Omer et al., Plant Growth Regul. 43:93-96 (2004)), enzymes such as pyruvate decarboxylase and alcohol dehydrogenase to generate ethanol (Deng and Coleman, Appl. Environ. Microbiol. 65:523-528 (1999)), butanol production from crotonyl-CoA via expression of enzymes such as Bcd, EtfAB, and AdhE2 from *Clostridium acetobutylicum* (Shen et al., Appl. Environ. Microbiol. 77:2905-2915 (2011)), or 1,4-butanediol from succinyl-CoA or α-ketoglutarate via CoA-dependent succinate semialdehyde dehydrogenase or 2-oxoglutarate decarboxylase, then 4-hydroxybutyrate dehydrogenase, 4-hydroxybutyrl-CoA transferase, 4-hydroxybutyryl-CoA reductase, and alcohol dehydrogenase (Yim et al., Nat. Chem. Biol. 7:445-452 (2011)). Protein production can be accomplished via strong expression systems for methylotrophic bacteria, such as those dependent upon the methanol dehydrogenase promoter (P*_{mxaF}*) of *M. extorquens* AMI such as pCM80, pCM110, pCM160 (Marx and Lidstrom, *Microbiology* 147-2065-2075 (2001)), or inducer-regulated versions such as pHC112 (Chou and Marx, Cell Reports 1:1-8 (2012)).

### EXAMPLE I

### RuMP Pathway for C₁ metabolism

The Ribulose Monophosphate pathway for C₁ metabolism is present in several methylotrophs such as the obligate methylotroph *Methylobacillus flagellatus* KT and the methanotroph *Methylococcus capsulatus* Bath. Methylotrophs growing on methanol using the RuMP Pathway assimilate carbon at the oxidation level of formaldehyde in comparison to formate, which is the intermediate that gets assimilated in methylotrophs using the serine cycle. Formaldehyde is condensed with ribulose monophosphate by an enzyme that is unique to this pathway called hexulose-6-phosphate synthase (HPS) to produced hexulose 6-phosphate. Hexulose 6-phosphate is isomerized into fructose 6-phophate by another enzyme that is unique to this pathway called hexulose 6-phophate isomerase (PHI). The fructose 6-phosphate can be assimilated either with certain enzymes that are involved in the pentose phosphate pathway or with certain enzymes that are involved in the Entner-Doudoroff pathway depending on the organism as listed in Table 1. If the fructose 6-phosphate is assimilated through the pentose phosphate pathway then two molecules of fructose 6-phophate are converted to two molecules of fructose bisphosphate each of which is cleaved to give four molecules of triose phosphate in total. One of these four molecules of triose phosphate is assimilated into biomass by conversion to pyruvate, which leads to the production of one NADPH and one ATP. The three molecules of triose phosphate are rearranged with one more fructose 6-phophate molecule through a serious of reactions with transketolase, transaldolase/sedoheptulose phosphatase, and finally pentose phosphate isomerases to regenerate three molecules of ribulose monophosphate as listed in Table 1. If the fructose 6-phosphate is assimilated through enzymes of the Entner-Doudoroff pathway then one molecule of fructose 6-phosphate is cleaved by a series of reactions to pyruvate and a triose phosphate. The triose phosphate along with two other molecules of fructose 6-phosphate are rearranged through a series of reactions with transketolase, transaldolase/sedoheptulose phosphatase, and finally pentose phosphate isomerases to regenerate three molecules of ribulose monophosphate as listed in Table 1. The RuMP pathway used for the assimilation of C₁ compounds with fructose bis-phosphate aldolase (an enzyme also involved in the pentose phosphate pathway) and transaldolase as the key enzymes involved in cleavage and rearrangement respectively is extremely efficient. It is to be understood that although some bacteria may require only HPS and PHI, other bacteria may include additional enzymes or directed alteration of their expression. For one molecule of pyruvate produced 1 molecule of NADPH and 1 molecule of ATP are also produced. On the contrary, the serine cycle leads to the net consumption of 1 molecule of NADPH and 1 molecule of ATP per molecule of pyruvate produced.

The Ribulose monophosphate pathway can also be used for the dissimilation of C₁ compounds. The fructose 6-phosphate generated by HPS and PHI is isomerized to glucose monophosphate by a hexose phosphate isomerase. Glucose monophosphate is oxidized to 6-phosphogluconate coupled with the reduction of NADP⁺ to NADPH. The 6-phosphogluconate thus formed is oxidized to ribulose monophosphate with the release of CO₂ and reduction of another molecule of NADP⁺ to NADPH by an enzyme called phosphogluconate dehydrogeanse as listed in Table 1.

While the RuMP pathway produces 2 molecules of NADPH per molecule of formaldehyde dissimilated, the H₄MPT pathway produces 1 molecule of NADPH and ∼1 molecule of NADH per molecule of formaldehyde dissimilated. Purely thinking in terms of energetics of dissimilation, neither one has a higher energy output per unit carbon. However, the RuMP pathway for dissimilation of C₁ compounds involves fewer enzymes and does not require a complex cofactor like H₄MPT that is used by the H₄MPT dissimilatory pathway for C₁ compounds.

### EXAMPLE II

### RuMP Pathway for C₁ metabolism: Strain 1

Strain 1- Replacing the native H₄MPT (tetrahydromethanopterin) pathway for C₁ dissimilation with the RuMP pathway. The serine cycle will be kept intact. However, since no formate will be produced during dissimilation of C₁ by the RuMP pathway, the serine cycle will become redundant and the RuMP pathway will take over C₁ assimilation as well as shown in Figure 2.

As described above, the RuMP pathway and the H₄MPT pathway are almost equivalent in terms of the net energetic output per molecule of methanol completely oxidized to CO₂. However, taking into account the fact that there are fewer enzymes involved in C₁ dissimilation using the RuMP pathway than the H₄MPT pathway as well as incorporating the cost of production of the complex cofactor in the H₄MPT pathway, we postulate that the RuMP pathway might carry out dissimilation of C₁ compounds more efficiently. Dissimilation of C₁ compounds through the introduced dissimilatory RuMP pathway, however, cannot be coupled to the native serine cycle for assimilation. This is because the key metabolic intermediate that gets fed into the serine cycle - formate - is not produced during dissimilation of methanol through the RuMP pathway. Hence, despite being intact in strain 1, the serine cycle should play a very minor role in assimilation of C₁ compounds and flux to biomass should mainly be routed through the RuMP pathway as well. Strain 1 was constructed by eliminating the native H₄MPT pathway for C₁ dissimilation by deleting a gene involved in the biosynthesis of the cofactor - tetrahydromethanopterin- and replacing it with a genes encoding hexulose 6-phosphate synthase (HPS) as well as a hexulose 6-phosphate isomerase (PHI) from *M. capsulatus* Bath. It should be noted that only two genes - those encoding HPS and PHI- are needed in the *Methylobacterium* genome to allow the RuMP pathway to dissimilate C₁ compounds since all other genes in this pathway are already present in the host strain.

The strain was made with a construct as shown in Figure 5. The construct was generated as follows. The specific gene in the biosynthetic pathway of tetrahydromethanopterin that was knocked out is *mptG* (Mext_1828 on the *M. extorquens* PA1 chromosome, ref) which is a ribofuranosylaminobenzene 5'-phosphate (RFAP) synthase (Chistoserdova et al., J. Bacteriol. 187:2508-2512 (2005)). The ORF encoding *mptG* is 1023 bases long and is found on the positive strand between bases 2049113 and 2050135 on the chromosome. The upstream flank of *mptG* for the construct (2048710-2049237) was amplified by a forward primer containing the recognition sequence for the restriction enzyme *Xba*I at the 5' end (ATCTAGACTTCCTCACCGTCGCTTCTTCG) and a reverse primer containing the recognition sequence for the restriction enzyme *Nde*I at the 5' end (TCATATGCGTTGAGATCGAGGAAGCCGA). The downstream flank of *mptG* for the construct (2049864-2050304) was amplified by a forward primer containing the recognition sequence for the restriction enzyme *Apa*I at the 5' end (ACTCGAGGCATCACCGCGATCCAGAAGC) and a reverse primer containing the recognition sequence for the restriction enzyme *Sac*I at the 5' end (TGAGCTCACGTCGAACGGGCTCATGT).

On investigating the genome sequence of *M. extorquens* PA1, it was evident that the only genes needed to introduce the dissimilatory RuMP cycle in the *M. extorquens* PA1 metabolic network were HPS and PHI. These genes were introduced from *M. capsulatus* Bath. In *M. capsulatus* Bath there are three genes encoding a HPS (MCA2738, MCA3043, and MCA3049 on the main chromosome of *Me. capsulatus* Bath), as well as three genes encoding a PHI (MCA2738, MCA3044, and MCA3050 on the main chromosome of *M. capsulatus* Bath). Previous work (Ward et al., PLoS Biology 2:e303 (2004)) has shown that most carbon flux enters the RUMP cycle through MCA3049 and MCA3050. Hence, these two genes were introduced in place of the *mptG* gene in *M. extorquens* PA1 for making Strain1. As stated above, MCA3049 encodes a hexulose 6-phosphate synthase (HPS) gene that is 648 bp long on the positive strand from position 3236945 to position 3237592 on the chromosome. MCA3050 encodes the hexulose 6-phosphate isomerase (PHI) gene that is 534 bp long on the positive strand from position 3237605 to position 3238138 on the chromosome. A forward primer with a recognition sequence for the restriction enzyme *Nde*I at the 5' end (TCATATGACCGGTGTCGCCAGGTACA) and a reverse primer with a recognition sequence for the restriction enzyme *Apa*I at the 5' end (TGGGCCCACGGACAACATGCCACGC) were used to amplify a region between position 3236701 and 3238196 on the *M. capsulatus* Bath chromosome containing the promoter, the RBS (ribosome binding site) as well as the as the entire ORF for MCA3049 and MCA 3050.

The final construct was made on the backbone of an allelic exchange vector (pCM433), as described in (Marx, BMC Research Notes 1:1 (2008)), in the order described below. The vector pCM433 was digested with restriction enzymes *Nde*I and *Xba*I*,* as was the insert, the *mptG* upstream flank. The 5' end of the digested vector was dephosphorylated using an Antarctic Phosphatase manufactured by NEB (New England Biolabs). The digested vector and insert were gel extracted using the Gel Extraction kit manufactured by Zymo Research Inc. A 3:1 molar ratio of the insert to vector was ligated using the Quick Ligase Kit manufactured by NEB (New England Biolabs). 1 µl of the ligated mixture was transformed in 50 µl of 10β chemically competent cells manufactured by NEB (New England Biolabs). The transformed cells were plated on LB + tetracycline (12.5 µg/ml) + 1.2% agar plates and screened by PCR for the insert. Colonies that screened positive for the insert were grown in LB+ tetracycline (12.5 µg/ml). The resulting plasmid with the *mptG* upstream flank in pCM433 was named pDN117. Plasmid extraction was done using the standard Miniprep kit manufactured by Qiagen. For the next round, a *dam⁻*/*dcm⁻* version of pDN117 was used as the vector and a *dam⁻*/*dcm⁻* version of the pCRII- Blunt- Topo vector containing *mptG* downstream flank as the insert. The same protocol described above was repeated. The resulting plasmid was named pDN128. For the final round, a *dam⁻*/*dcm⁻* version of pDN128 was used as the vector and a *dam⁻*/*dcm⁻* version of the pCRII- Blunt- Topo vector with the region of the *Methylococcus capsulatus* Bath containing the MCA3049 and MCA3050 ORF, promoter and RBS as the insert and the same protocol described above was repeated. The resulting plasmid was named pDN131. All plasmids generated were verified by Sanger Sequencing and an *E. coli* strain containing each plasmid was cryopreserved with 10% DMSO in a -80 °C freezer.

The final construct generated (pDN131) was introduced in a Δ*cel M. extorquens* PA1 strain using a three-way mating protocol. The *E. coli* strain containing pDN131 was grown in 10 ml of LB + tetracycline (12.5 µg/ml), a strain containing the a helper plasmid with the conjugal transfer genes of the broad host-range plasmid RK2 called pRK2073 was grown 10ml in LB + Streptomycin (50.0 µg/ml), and *M. extorquens* PA1 was grown in 10 ml Hypho minimal media with 3.5 mM succinate as the carbon source. The *E. coli* strains were harvested in early stationary phase and *M. extorquens* PA1 was harvested in mid-exponential phase (OD₆₀₀ ∼0.3). Centrifuged *E. coli* cells were resuspended in 1 ml of fresh LB media without any antibiotic. Centrifuged *M. extorquens* PA1 cells were resuspended in 1 ml of Hypho minimal media. 50 µl of the pRK2073 resuspended in LB was added to the resuspended *E. coli* strain with pDN131 and incubated in a 37 °C shaking incubator for one hour. 10 µl of this culture was spotted on a Nutrient Agar plate and allowed to air dry. 50 µl of the *M. extorquens* PA1 culture was spotted on top of the *E. coli* culture on the Nutrient Agar plate and allowed to air dry. The plate was incubated at 30 °C to overnight and at 4 °C for a day. The colony growth was suspended in 100 µl of fresh hypho and plated on a hypho + 3.5 mM succinate + 1.2% agar + tetracycline (10 µg/ml) minimal media plate. Colonies from the first tetracycline plate were streaked on another hypho + 3.5 mM succinate + 1.2% agar + tetracycline (10 µg/ml) minimal media plate to ensure tetracycline resistance. Tetracycline resistant colonies are those that had integrated the allelic exchange vector in the chromosome. These colonies were then grown in hypho + 3.5 mM succinate overnight without any antibiotic. The overnight growth was plate on hypho + succinate + 1.2% agar + 5% sucrose minimal media plates. Since sucrose is a counter-selection agent, this step was used to select for colonies that had excised the allelic exchange vector out of the chromosome through homologous recombination. Unintended mutational inactivation of the *sacB* (responsible for sucrose sensitivity) gene on the allelic exchange vector was screened out by testing the sucrose⁺ colonies for tetracycline resistance. Those colonies that had a sucrose+ and tetracycline sensitive phenotype were tested for the ancestral or modified version of the chromosome at the *mptG* locus using PCR. Colonies that had replaced the *mptG* locus with the HPS and PHI genes of the RuMP pathway were verified by Sanger Sequencing and cyro-preserved in a -80 °C freezer with 10% DMSO.

### EXAMPLE III

### RuMP Pathway for C₁ metabolism: Strain 2

Strain 2- Replacing the native serine cycle for C₁ assimilation with the RuMP pathway. The H₄MPT pathway for C₁ dissimilation will be kept intact however, due to the absence of a key gene in the serine cycle, carbon will not be assimilated from the formate produced during dissimilation as shown in Figure 3.

As described above the RuMP pathway used for the assimilation of C₁ compounds with fructose bis-phosphate aldolase (an enzyme also involved in the pentose phosphate pathway) and transaldolase as the key enzymes involved in cleavage and rearrangement respectively is extremely efficient. For one molecule of pyruvate produced 1 molecule of NADPH and 1 molecule of ATP are also produced. On the contrary, the serine cycle leads to the net consumption of 1 molecule of NADPH and 1 molecule of ATP per molecule of pyruvate produced as well as production of increased CO₂ per unit substrate. Hence replacing the native serine cycle in *Methylobacterium* with the RuMP pathway will not only increase the efficiency of carbon assimilation but also the amount of carbon assimilated into biomass per unit substrate. Strain 2 was constructed by eliminating the native serine cycle for C₁ assimilation by deleting a gene involved in serine cycle and replacing it with a genes encoding hexulose 6-phosphate synthase (HPS) as well as a hexulose 6-phosphate isomerase (PHI) from *M. capsulatus* Bath. It should be noted that only two genes - those encoding HPS and PHI- are needed in the *Methylobacterium* genome to allow the RuMP pathway to assimilate C₁ compounds since all other genes in this pathway are already present in the host strain.

The construct used for the generation of this strain is shown in Figure 6. The construct was made as follows. The specific gene in the serine cycle that was knocked out is *hprA* (Mext_1796 on the *M. extorquens* PA1 chromosome, ref), which is an NADPH dependent hydroxypyruvate reductase described in (Chistoserdova et al., J. Bacteriol. 185(10): 2980-2987 (2003)). The ORF encoding *hprA* is 945 bases long and is found on the positive strand between bases 20161384 and 2017078 on the chromosome. The upstream flank of *hprA* for the construct (2015764-2016107) was amplified by a forward primer containing the recognition sequence for the restriction enzyme *Xba*I at the 5' end (TCTAGACTC ATC GAC AAC GGC GTG AAG G) and a reverse primer containing the recognition sequence for the restriction enzyme *Nde*I at the 5' end (CATATGGGGCAATCGTGTCGCTCAC). The downstream flank of *mptG* for the construct (2017019-2017465) was amplified by a forward primer containing the recognition sequence for the restriction enzyme *Apa*I at the 5' end (GGGCCCCTCGTGGACAACGTCGAAGC) and a reverse primer containing the recognition sequence for the restriction enzyme *Sac*I at the 5' end (GAGCTCTCT TCA CCG CCT CGA ACA CC).

On investigating the genome sequence of *M. extorquens* PA1, it was evident that the only genes needed to introduce the dissimilatory RuMP cycle in the *M. extorquens* PA1 metabolic network were HPS and PHI. These genes were introduced from *M. capsulatus* Bath. In *M. capsulatus* Bath there are three genes encoding a HPS (MCA2738, MCA3043, and MCA3049 on the main chromosome of *Me. capsulatus* Bath), as well as three genes encoding a PHI (MCA2738, MCA3044, and MCA3050 on the main chromosome of *M. capsulatus* Bath). Previous work (Ward et al., PLoS Biology 2:e303 (2004)) has shown that most carbon flux enters the RUMP cycle through MCA3049 and MCA3050. Hence, these two genes were introduced in place of the *mptG* gene in *M. extorquens* PA1 for making Strain1. As stated above, MCA3049 encodes a hexulose 6-phosphate synthase (HPS) gene that is 648 bp long on the positive strand from position 3236945 to position 3237592 on the chromosome. MCA3050 encodes the hexulose 6-phosphate isomerase (PHI) gene that is 534 bp long on the positive strand from position 3237605 to position 3238138 on the chromosome. A forward primer with a recognition sequence for the restriction enzyme *Nde*I at the 5' end (TCATATGACCGGTGTCGCCAGGTACA) and a reverse primer with a recognition sequence for the restriction enzyme *Apa*I at the 5' end (TGGGCCCACGGACAACATGCCACGC) were used to amplify a region between position 3236701 and 3238196 on the *M. capsulatus* Bath chromosome containing the promoter, the RBS (ribosome binding site) as well as the as the entire ORF for MCA3049 and MCA 3050.

The final construct was made on the backbone of an allelic exchange vector (pCM433), as described in (Marx, BMC Research Notes 1:1 (2008)), in the order described below. The vector pCM433 was digested with restriction enzymes *Nde*I and *Xba*I*,* as was the insert, the *hprA* upstream flank. The 5' end of the digested vector was dephosphorylated using an Antarctic Phosphatase manufactured by NEB (New England Biolabs). The digested vector and insert were gel extracted using the Gel Extraction kit manufactured by Zymo Research Inc. A 3:1 molar ratio of the insert to vector was ligated using the Quick Ligase Kit manufactured by NEB (New England Biolabs). 1 µl of the ligated mixture was transformed in 50 µl of 10β chemically competent cells manufactured by NEB (New England Biolabs). The transformed cells were plated on LB + tetracycline (12.5 µg/ml) + 1.2% agar plates and screened by PCR for the insert. Colonies that screened positive for the insert were grown in LB+ tetracycline (12.5 µg/ml). The resulting plasmid was named pDN123. Plasmid extraction was done using the standard Miniprep kit manufactured by Qiagen. For the next round, a *dam⁻*/*dcm⁻* version of pDN123 was used as the vector and a *dam⁻*/*dcm⁻* version of the pCRII- Blunt- Topo vector containing *hprA* downstream flank as the insert. The same protocol described above was repeated. The resulting plasmid was named pDN127. For the final round, a *dam⁻*/*dcm⁻* version of pDN127 was used as the vector and a *dam⁻*/*dcm⁻* version of the pCRII- Blunt- Topo vector with the region of the *Methylococcus capsulatus* Bath containing the MCA3049 and MCA3050 ORF, promoter and RBS as the insert and the same protocol described above was repeated. The resulting plasmid was named pDN132. All plasmids generated were verified by Sanger Sequencing and an *E. coli* strain containing each plasmid was cryopreserved with 10% DMSO in a -80 °C freezer.

The final construct generated (pDN132) was introduced in a Δ*cel M. extorquens* PA1 strain using a three-way mating protocol. The *E. coli* strain containing pDN132 was grown in 10 ml of LB + tetracycline (12.5 µg/ml), a strain containing the a helper plasmid with the conjugal transfer genes of the broad host-range plasmid RK2 called pRK2073 was grown 10 ml in LB + streptomycin (50.0 µg/ml), and *M. extorquens* PA1 was grown in 10 ml Hypho minimal media with 3.5 mM succinate as the carbon source. The *E. coli* strains were harvested in early stationary phase and *M. extorquens* PA1 was harvested in mid-exponential phase (OD₆₀₀ ∼0.3). Centrifuged *E. coli* cells were resuspended in 1 ml of fresh LB media without any antibiotic. Centrifuged *M. extorquens* PA1 cells were resuspended in 1 ml of Hypho minimal media. 50 µl of the pRK2073 resuspended in LB was added to the resuspended *E. coli* strain with pDN131 and incubated in a 37 °C shaking incubator for one hour. 10 µl of this culture was spotted on a Nutrient Agar plate and allowed to air dry. 50 µl of the *M. extorquens* PA1 culture was spotted on top of the *E.coli* culture on the Nutrient Agar plate and allowed to air dry. The plate was incubated at 30 °C to overnight and at 4 °C for a day. The colony growth was suspended in 100 µl of fresh hypho and plated on a hypho + 3.5 mM succinate + 1.2% agar + tetracycline (10 µg/ml) minimal media plate. Colonies from the first tetracycline plate were streaked on another hypho + 3.5 mM succinate + 1.2% agar + tetracycline (10 µg/ml) minimal media plate to ensure tetracycline resistance. Tetracycline resistant colonies are those that had integrated the allelic exchange vector in the chromosome. These colonies were then grown in hypho + 3.5 mM succinate overnight without any antibiotic. The overnight growth was plate on hypho + succinate + 1.2% agar + 5% sucrose minimal media plates. Since sucrose is a counter-selection agent, this step was used to select for colonies that had excised the allelic exchange vector out of the chromosome through homologous recombination. Unintended mutational inactivation of the *sacB* (responsible for sucrose sensitivity) gene on the allelic exchange vector was screened out by testing the sucrose⁺ colonies for tetracycline resistance. Those colonies that had a sucrose+ and tetracycline sensitive phenotype were tested for the ancestral or modified version of the chromosome at the *hprA* locus using PCR. Colonies that had replaced the *hprA* locus with the HPS and PHI/PHI genes of the RuMP pathway were verified by Sanger Sequencing and cyro-preserved in a -80 °C freezer with 10% DMSO.

### EXAMPLE IV

### RuMP Pathway for C₁ metabolism: Strain 3

Strain 3 - Replacing the native H₄MPT pathway for C₁ dissimilation and the native serine cycle for C₁ assimilation with the RuMP pathway. This engineered strain should solely use the RuMP pathway to grow on methanol as shown in Figure 4.

As described above the RuMP pathway used for the assimilation of C₁ compounds with fructose bis-phosphate aldolase (an enzyme also involved in the pentose phosphate pathway) and transaldolase as the key enzymes involved in cleavage and rearrangement respectively is extremely efficient. For one molecule of pyruvate produced 1 molecule of NADPH and 1 molecule of ATP are also produced. On the contrary, the serine cycle leads to the net consumption of 1 molecule of NADPH and 1 molecule of ATP per molecule of pyruvate produced as well as production of increased CO₂ per unit substrate. Hence replacing the native serine cycle in *Methylobacterium* with the RuMP pathway will not only increase the efficiency of carbon assimilation but also the amount of carbon assimilated into biomass per unit substrate. Similarly, taking into account the fact that there are fewer enzymes involved in C₁ dissimilation using the RuMP pathway than the H₄MPT pathway as well as incorporating the cost of production of the complex cofactor in the H₄MPT pathway, we postulate that the RuMP pathway might carry out dissimilation of C₁ compounds more efficiently. Hence a strain of *Methylobacterium* that uses the RuMP pathway for assimilation and dissimilation of C₁ compounds might be able to growth faster, assimilate carbon more efficiently and also channel more carbon to biomass and reduce CO₂ production per unit substrate.

Strain 3 was constructed by first deleting the H₄MPT (tetrahydromethanopterin) pathway for C₁ dissimilation in *Methylobacterium* and then eliminating the native serine cycle for C₁ assimilation by deleting a gene involved in serine cycle and replacing it with a genes encoding hexulose 6-phosphate synthase (HPS) as well as a hexulose 6-phosphate isomerase (PHI) from *M. capsulatus* Bath. It should be noted that only two genes - those encoding HPS and PHI- are needed in the *Methylobacterium* genome to allow the RuMP pathway to assimilate C₁ compounds since all other genes in this pathway are already present in the host strain.

The construct for deleting *mptG* is shown in Figure 7. The specific gene in the biosynthetic pathway of tetrahydromethanopterin that was knocked out is *mptG* (Mext_1828 on the *M. extorquens* PA1 chromosome), which is a ribofuranosylaminobenzene 5'-phosphate (RFAP) (Chistoserdova et al., J. Bacteriol. 187:2508-2512 (2005)). The ORF encoding *mptG* is 1023 bases long and is found on the positive strand between bases 2049113 and 2050135 on the chromosome. A forward primer containing a 30 bp overhang (ATGGATGCATATGCTGCAGCTACGAGCGGCCGCGAT CTC GGC GAT CAG CTC ACC) corresponding to bases 1029 through 1060 on the positive strand of pCM433 and a reverse primer (GCC GTT GAG ATC GAG GAA GCC) were used to amplify the upstream flank from position 2048391 to 2049259 on the *M. extorquens* PA1 chromosome. A forward primer containing a 30 bp overhang (CTGCATTTCGGCTTCCTCGATCTCAACGGCCTC GCA GAA GGA GGC GGA) spanning the region from 2049230 through 2049259 on the positive strand of the *M. extorquens* PA1 chromosome and a reverse primer (GGTTAACACGCGTACGTAGGGCCCGCGGCCGCGGT GAA GGC GAT CTT CGA GAC G) with a 30 bp overhang spanning the region from 1053 to 1084 on the negative strand of pCM433 was used to amplify the downstream flank from position 2050027 to 2050749 on the *M.extorquens* PA1 chromosome. The final construct was made on the backbone of the pCM433 allelic exchange vector (Marx, BMC Research Notes 1:1 (2008)) using the protocol described in (Gibson et al., Nature Methods 6:343-345(2009)). The construct containing the upstream and downstream flanks of *mptG* was named pDN68 and verified by Sanger sequencing and cryo-preserved in a NEB10β competent *E. coli* strain with 10% DMSO in a -80 °C freezer. pDN68 was introduced in a Δ*cel M. extorquens* PA1 strain using a three-way mating protocol. The *E. coli* strain containing pDN168was grown in 10 ml of LB + tetracycline (12.5 µg/ml), a strain containing the a helper plasmid with the conjugal transfer genes of the broad host-range plasmid RK2 called pRK2073 was grown 10 ml in LB + Streptomycin (50.0 µg/ml), and *M. extorquens* PA1 was grown in 10 ml Hypho minimal media with 3.5 mM succinate as the carbon source. The *E. coli* strains were harvested in early stationary phase and *M. extorquens* PA1 was harvested in mid-exponential phase (OD₆₀₀ ∼0.3). Centrifuged *E. coli* cells were resuspended in 1 ml of fresh LB media without any antibiotic. Centrifuged *M. extorquens* PA1 cells were resuspended in 1 ml of Hypho minimal media. 50 µl of the pRK2073 resuspended in LB was added to the resuspended *E. coli* strain with pDN131 and incubated in a 37 °C shaking incubator for one hour. 10 µl of this culture was spotted on a Nutrient Agar plate and allowed to air dry. 50 µl of the *M. extorquens* PA1 culture was spotted on top of the *E. coli* culture on the Nutrient Agar plate and allowed to air dry. The plate was incubated at 30 °C to overnight and at 4 °C for a day. The colony growth was suspended in 100 µl of fresh hypho and plated on a hypho + 3.5 mM succinate + 1.2% agar + tetracycline (10 µg/ml) minimal media plate. Colonies from the first tetracycline plate were streaked on another hypho + 3.5 mM succinate + 1.2% agar + tetracycline (10 µg/ml) minimal media plate to ensure tetracycline resistance. Tetracycline resistant colonies are those that had integrated the allelic exchange vector in the chromosome. These colonies were then grown in hypho + 3.5 mM succinate overnight without any antibiotic. The overnight growth was plate on hypho + succinate + 1.2% agar + 5% sucrose minimal media plates. Since sucrose is a counter-selection agent, this step was used to select for colonies that had excised the allelic exchange vector out of the chromosome through homologous recombination. Unintended mutational inactivation of the *sacB* (responsible for sucrose sensitivity) gene on the allelic exchange vector was screened out by testing the sucrose⁺ colonies for tetracycline resistance. Those colonies that had a sucrose+ and tetracycline sensitive phenotype were tested for the ancestral or knockout version of the chromosome at the *mptG* locus using PCR. Colonies that deleted the *mptG* locus were verified by Sanger Sequencing and cyro-preserved in a -80° C freezer with 10% DMSO.

The specific gene in the serine cycle that was knocked out is *hprA* (Mext_1796 on the *M. extorquens* PA1 chromosome, ref), which is a NADPH dependent hydroxypyruvate reductase, which is described in (Chistoserdova et al., J. Bacteriol. 185(10): 2980-2987 (2003)). The ORF encoding *hprA* is 945 bases long and is found on the positive strand between bases 20161384 and 2017078 on the chromosome. The upstream flank of *hprA* for the construct (2015764-2016107) was amplified by a forward primer containing the recognition sequence for the restriction enzyme *Xba*I at the 5' end (TCTAGACTC ATC GAC AAC GGC GTG AAG G) and a reverse primer containing the recognition sequence for the restriction enzyme *Nde*I at the 5' end (CATATGGGGCAATCGTGTCGCTCAC). The downstream flank of *mptG* for the construct (2017019-2017465) was amplified by a forward primer containing the recognition sequence for the restriction enzyme *Apa*I at the 5' end (GGGCCCCTCGTGGACAACGTCGAAGC) and a reverse primer containing the recognition sequence for the restriction enzyme *Sac*I at the 5' end (GAGCTCTCT TCA CCG CCT CGA ACA CC).

On investigating the genome sequence of *M. extorquens* PA1, it was evident that the only genes needed to introduce the dissimilatory RuMP cycle in the *M. extorquens* PA1 metabolic network were HPS and PHI. These genes were introduced from *M. capsulatus* Bath. In *M. capsulatus* Bath there are three genes encoding a HPS (MCA2738, MCA3043, and MCA3049 on the main chromosome of *Me. capsulatus* Bath), as well as three genes encoding a PHI (MCA2738, MCA3044, and MCA3050 on the main chromosome of *M. capsulatus* Bath). Previous work (Ward et al., PLoS Biology 2:e303 (2004))has shown that most carbon flux enters the RUMP cycle through MCA3049 and MCA3050. Hence, these two genes were introduced in place of the *mptG* gene in *M. extorquens* PA1 for making Strain1. As stated above, MCA3049 encodes a hexulose 6-phosphate synthase (HPS) gene that is 648 bp long on the positive strand from position 3236945 to position 3237592 on the chromosome. MCA3050 encodes the hexulose 6-phosphate isomerase (PHI) gene that is 534 bp long on the positive strand from position 3237605 to position 3238138 on the chromosome. A forward primer with a recognition sequence for the restriction enzyme *Nde*I at the 5' end (TCATATGACCGGTGTCGCCAGGTACA) and a reverse primer with a recognition sequence for the restriction enzyme *Apa*I at the 5' end (TGGGCCCACGGACAACATGCCACGC) were used to amplify a region between position 3236701 and 3238196 on the *M. capsulatus* Bath chromosome containing the promoter, the RBS (ribosome binding site) as well as the as the entire ORF for MCA3049 and MCA 3050.

The final construct was made on the backbone of an allelic exchange vector (pCM433), as described in (Marx, BMC Research Notes 1:1 (2008)), in the order described below. The vector pCM433 was digested with restriction enzymes *Nde*I and *Xba*I*,* as was the insert, the *hprA* upstream flank. The 5' end of the digested vector was dephosphorylated using an Antarctic Phosphatase manufactured by NEB (New England Biolabs). The digested vector and insert were gel extracted using the Gel Extraction kit manufactured by Zymo Research Inc. A 3:1 molar ratio of the insert to vector was ligated using the Quick Ligase Kit manufactured by NEB (New England Biolabs). 1 µl of the ligated mixture was transformed in 50 µl of 10β chemically competent cells manufactured by NEB (New England Biolabs). The transformed cells were plated on LB + tetracycline (12.5 µg/ml) + 1.2% agar plates and screened by PCR for the insert. Colonies that screened positive for the insert were grown in LB+ tetracycline (12.5 µg/ml). The resulting plasmid was named pDN123. Plasmid extraction was done using the standard Miniprep kit manufactured by Qiagen. For the next round, a *dam⁻*/*dcm⁻* version of pDN123 was used as the vector and a *dam⁻*/*dcm⁻* version of the pCRII- Blunt- Topo vector containing *hprA* downstream flank as the insert. The same protocol described above was repeated. The resulting plasmid was named pDN127. For the final round, a *dam⁻*/*dcm⁻* version of pDN127 was used as the vector and a *dam⁻*/*dcm⁻* version of the pCRII- Blunt- Topo vector with the region of the *Methylococcus capsulatus* Bath containing the MCA3049 and MCA3050 ORF, promoter and RBS as the insert and the same protocol described above was repeated. The resulting plasmid was named pDN132. All plasmids generated were verified by Sanger Sequencing and an *E. coli* strain containing each plasmid was cryopreserved with 10% DMSO in a -80 °C freezer.

The final construct generated (pDN132) was introduced in a Δ*cel* Δ*mptG M. extorquens* PA1 strain using a three-way mating protocol. The *E. coli* strain containing pDN132 was grown in 10 ml of LB + tetracycline (12.5 µg/ml), a strain containing the a helper plasmid with the conjugal transfer genes of the broad host-range plasmid RK2 called pRK2073 was grown 10 ml in LB + Streptomycin (50.0 µg/ml), and *M. extorquens* PA1 was grown in 10 ml Hypho minimal media with 3.5 mM succinate as the carbon source. The *E. coli* strains were harvested in early stationary phase and *M. extorquens* PA1 was harvested in mid-exponential phase (OD₆₀₀ ∼0.3). Centrifuged *E. coli* cells were resuspended in 1 ml of fresh LB media without any antibiotic. Centrifuged *M. extorquens* PA1 cells were resuspended in 1 ml of Hypho minimal media. 50 µl of the pRK2073 resuspended in LB was added to the resuspended *E. coli* strain with pDN131 and incubated in a 37 °C shaking incubator for one hour. 10 µl of this culture was spotted on a Nutrient Agar plate and allowed to air dry. 50 µl of the *M. extorquens* PA1 culture was spotted on top of the *E.coli* culture on the Nutrient Agar plate and allowed to air dry. The plate was incubated at 30 °C to overnight and at 4 °C for a day. The colony growth was suspended in 100 µl of fresh hypho and plated on a hypho + 3.5 mM succinate + 1.2% agar + tetracycline (10 µg/ml) minimal media plate. Colonies from the first tetracycline plate were streaked on another hypho + 3.5 mM succinate + 1.2% agar + tetracycline (10 µg/ml) minimal media plate to ensure tetracycline resistance. Tetracycline resistant colonies are those that had integrated the allelic exchange vector in the chromosome. These colonies were then grown in hypho + 3.5 mM succinate overnight without any antibiotic. The overnight growth was plate on hypho + succinate + 1.2% agar + 5% sucrose minimal media plates. Since sucrose is a counter-selection agent, this step was used to select for colonies that had excised the allelic exchange vector out of the chromosome through homologous recombination. Unintended mutational inactivation of the *sacB* (responsible for sucrose sensitivity) gene on the allelic exchange vector was screened out by testing the sucrose⁺ colonies for tetracycline resistance. Those colonies that had a sucrose+ and tetracycline sensitive phenotype were tested for the ancestral or modified version of the chromosome at the *hprA* locus using PCR. Colonies that had replaced the *hprA* locus with the HPS and PHI genes of the RuMP pathway were verified by Sanger Sequencing and cyro-preserved in a -80 °C freezer with 10% DMSO.

### EXAMPLE V

### Subsequent evolution to improve growth properties

### of strains resulting from Examples II-IV

The above strains generate methylotrophs with improved yield on C₁ compounds due to less CO₂ production. This results from the stoichiometric advantage of incorporating all carbon at the level of formaldehyde, rather than from a combination of formate and CO₂ for the serine cycle, or purely CO₂ for autotrophs. However, cells can be further optimized. To further develop strains in which central metabolism is to be engineered to produce valuable chemicals, strains are first evolved in batch culture to select for increased growth rate. This follows precedent with *M. extorquens* AM1 where the H₄MPT-dependent C₁ dissimilation pathway was replaced with the analogous, but non-homologous glutathione-dependent pathway from *Paracoccus denitrificans* (Marx et al., J. Bacteriol. 185:7160-7168 (2003)). Subsequent evolution in methanol-containing media resulted in dramatic increases in growth (Chou et al., Science 332:1190-1192 (2011)). Much of the improvement occurred in the first couple hundred generations of these experiments, and the extent of improvement varied considerably across replicates (Lee and Marx, Genetics 193:943-952 (2013)). Briefly, the desired starting strain to be used as an ancestor is streaked for individual colonies, each of which is used to found an independent replicate population. Cultures are then periodically diluted (1/2*ⁿ*) into fresh medium containing the C₁ substrate of interest, such as methanol, resulting in *n* net generations per transfer. Samples of the remaining culture are cryopreserved to provide a living fossil record of the experiment. Populations or isolates from them can be screened for improved growth under the desired conditions.

## Claims

1. A recombinant bacterium cell
genetically modified to disrupt the cell's H₄MPT pathway for C₁ dissimilation and to include a recombinant RuMP pathway for C₁ dissimilation and/or genetically modified to disrupt the cell's serine cycle for C₁ assimilation and to include a recombinant RuMP pathway for C₁ assimilation.

2. The recombinant bacterium cell of claim 1 being a *Methylobacterium.*

3. The recombinant bacterium cell of claim 1 being a *Methylobacterium extorquens.*

4. The recombinant bacterium cell of claim 1 being a *Methylobacterium extorquens* PA1.

5. A method of making a genetically modified bacterium comprising
genetically modifying a target bacterium to disrupt the target bacterium's H₄MPT pathway for C₁ dissimilation and to include a recombinant RuMP pathway for C₁ dissimilation and/or genetically modifying a target bacterium to disrupt the target bacterium's serine cycle for C₁ assimilation and to include a recombinant RuMP pathway for C₁ assimilation.

6. The method of claim 5 wherein the target bacterium is a *Methylobacterium.*

7. The method of claim 5 wherein the target bacterium is a *Methylobacterium extorquens.*

8. The method of claim 5 wherein the target bacterium is a *Methylobacterium extorquens* PA1.

## Patentansprüche

1. Rekombinante Bakterienzelle
genetisch modifiziert, um den H₄MPT-Weg der Zelle für C₁-Dissimilation zu unterbrechen und einen rekombinanten RuMP-Weg für C₁-Dissimilation einzubeziehen, und/oder genetisch modifiziert, um den Serin-Zyklus der Zelle für C₁-Assimilation zu unterbrechen und einen rekombinanten RuMP-Weg für C₁-Assimilation einzubeziehen.

2. Rekombinante Bakterienzelle nach Anspruch 1, wobei es sich um ein *Methylobacterium* handelt.

3. Rekombinante Bakterienzelle nach Anspruch 1, wobei es sich um ein *Methylobacterium extorquens* handelt.

4. Rekombinante Bakterienzelle nach Anspruch 1, wobei es sich um ein *Methylobacterium extorquens* PA1 handelt.

5. Verfahren zur Herstellung eines genetischen modifizierten Bakteriums, umfassend
genetisches Modifizieren eines Zielbakteriums, um den H₄MPT-Weg des Zielbakteriums für C₁-Dissimilation zu unterbrechen und einen rekombinanten RuMP-Weg für C₁-Dissimilation einzubeziehen, und/oder genetisches Modifizieren eines Zielbakteriums, um den Serin-Zyklus des Zielbakteriums für C₁-Assimilation zu unterbrechen und einen rekombinanten RuMP-Weg für C₁-Assimilation einzubeziehen.

6. Verfahren nach Anspruch 5, wobei es sich bei dem Zielbakterium um ein *Methylobacterium* handelt.

7. Verfahren nach Anspruch 5, wobei es sich bei dem Zielbakterium um ein *Methylobacterium extorquens* handelt.

8. Verfahren nach Anspruch 5, wobei es sich bei dem Zielbakterium um ein *Methylobacterium extorquens* PA1 handelt.

## Revendications

1. Cellule bactérienne recombinante génétiquement modifiée pour rompre la voie de H₄MPT de la cellule pour une dissimilation de C₁ et pour inclure une voie de RuMP recombinante pour la dissimilation de C₁ et/ou génétiquement modifiée pour rompre le cycle de sérine de la cellule pour l'assimilation de C₁ et pour inclure une voie de RuMP recombinante pour l'assimilation de C₁.

2. Cellule bactérienne recombinante selon la revendication 1, qui est une *Methylobacterium.*

3. Cellule bactérienne recombinante selon la revendication 1, qui est une *Methylobacterium extorquens.*

4. Cellule bactérienne recombinante selon la revendication 1, qui est une *Methylobacterium extorquens* PA1.

5. Procédé de production d'une bactérie génétiquement modifiée, comprenant
la modification génétique d'une bactérie cible pour rompre la voie de H₄MPT de la bactérie cible pour une dissimilation de C₁ et pour inclure une voie de RuMP recombinante pour la dissimilation de C₁ et/ou la modification génétique d'une bactérie cible pour rompre le cycle de sérine de la bactérie cible pour l'assimilation de C₁ et pour inclure une voie de RuMP recombinante pour l'assimilation de C₁.

6. Procédé selon la revendication 5, dans lequel la bactérie cible est une *Methylobacterium.*

7. Procédé selon la revendication 5, dans lequel la bactérie cible est une *Methylobacterium extorquens.*

8. Procédé selon la revendication 5, dans lequel la bactérie cible est une *Methylobacterium extorquens* PA1.
